# EUROPEAN PATENT APPLICATION

(11) **EP 4 744 484 A1**
(43) Date of publication of application: **20.05.2026**
(21) Application number: 25215645.0
(22) Date of filing: 13.11.2025
(51) Int. Cl.: A01M 1/20

(54) **AN APPARATUS FOR ELIMINATING INSECTS OF GENUS CIMEX**

(30) Priority: 14.11.2024 FI 20246334
(71) Applicant: Otso3 Oy, 20660 Littoinen (FI)
(72) Inventor: Harjula, Kimmo, 20660 Littoinen (FI)
(74) Representative: Kolster Oy Ab

(57) **Abstract**

An apparatus (100) for eliminating insects of genus Cimex, the apparatus (100) comprises a heating device (1) and a pouch (3) that are separate units, the heating device (1) comprising a heating element (8) and a fan (9) inside a housing (18), the heating device (1) has an input (14) and an output (15) and the pouch (3) has an inlet (16) and an outlet (17), the inlet (16) of the pouch (3) being connectable to the output (15) of the heating device (1) and the outlet (17) of the pouch (3) being connectable to the input (14) of the heating device (1).

## Description

### FIELD OF THE INVENTION

The present invention relates to an apparatus for eliminating insects of genus Cimex.

### BACKGROUND OF THE INVENTION

Bedbugs are small wingless insects that suck human blood. Harms caused by the bedbugs have been increased in recent years. The bedbugs hide in utility articles, such as luggage, clothing and second-hand items, and spread in new environments with the utility articles. Globalization, climate change, growing tourism and increasing circular economy has speeded up spread of the bedbugs. Hotels, airports and flea markets provide good chances to the bedbugs to spread from one household to another by hiding in luggage or utility articles. Their presence causes physical discomfort, psychological stress and financial losses. Further, the population of the bedbugs increase steeply if they are allowed to reproduce freely.

There are a few of ways to get rid of the bedbugs. Different methods may be used alone or as a mixture of different methods. Insecticides are one possibility but commonly used insecticides, such as pyrethroids, have partly lost their effect because the bedbugs have created resistance against them through genetic mutations. Another way to eliminate the bedbugs is to use microorganisms or natural enemies of the bedbugs. Still another way is to use physical methods, such as heating or cooling the bedbugs to death. If properly made heating eliminates eggs, nymphs and adults.

### BRIEF DESCRIPTION OF THE INVENTION

An object of the present invention is to provide an apparatus for eliminating insects of genus Cimex. The objects of the invention are achieved by an apparatus which is characterized by what is stated in the independent claim. The preferred embodiments of the invention are disclosed in the dependent claims.

The invention is based on the idea of utilizing heat for eliminating insects of genus Cimex. The apparatus comprises a heating device and a pouch which are used for heat treatment where insects of genus Cimex are destroyed.

There are several advantages that relate to the apparatus. The apparatus is mobile and portable due to its compact size and two-part form, i.e. there are two separate units that form the apparatus, namely the heating device and the pouch.

The pouch is reusable, it may have different sizes and it may be changed according to a need. The pouch forms the space where the treatment takes place, i.e. there is a closed environment for the treatment.

The apparatus is energy efficient since it circulates air in a substantially closed system. Thus, the circulated air must be heated only to cover a heat loss that has taken place during its circle in the pouch.

The apparatus is easy to use and environmentally friendly since no chemicals are used and energy is used only as much as is necessary.

The apparatus is for eliminating insects of genus Cimex. Genus Cimex includes wingless insects, such as bedbugs (Cimex lectularius). The bedbugs are one of the most harmful species among genus Cimex. The apparatus may also be used for any other insect genus to whom a process performed in the apparatus is applicable.

The apparatus comprises a heating device and a pouch. It is possible that the apparatus consists of the heating device and the pouch. The heating device and the pouch are separate units, i.e. they can be connected to each other during use of the apparatus but they can be disconnected when the apparatus is not in use. The above-mentioned feature makes possible to store the apparatus in two different places, i.e. the heating device in one place and the pouch in another place. All in all, the apparatus requires a very small space which is important e.g. in households since the pouch is a flexible bag that is collapsible and can be flattened.

The heating device is a mains driven device adapted to work according to a local standard, such as 220 V - 240 V, 50 Hz or 100 V - 127 V, 60 Hz. The plug is naturally adapted to the local standard.

The heating device has a housing. There may be a starting switch on the housing. The starting switch may be a push button or a rocker switch. There may also be an indicator light on the housing that shows that the device is on. Instead of the indicator light there may be a display. The starting switch may be on the plug instead of the housing. The indicator light may also be on the plug.

The heating device comprises a heating element and a fan inside the housing. The heating element may be a thermal resistor. The heating element heats air that is circulated by the fan. The fan ensures a constant temperature and efficient heat transfer. An output of the heating device delivers heated air out of the device and an input of the heating device delivers air back to the device. The output has greater cross-sectional area for delivering air compared to that of the input. In other words, when the cross-sections of the input and output are circular the output has greater diameter compared to the input. Thus, the heating device is capable of creating positive pressure inside the pouch.

The cross-sectional area of the output may be 10 to 50 % greater than the cross-sectional area of the input. Preferably the cross-sectional area of the output may be 23 to 27 % greater than the cross-sectional area of the input.

There may be a temperature sensor in the vicinity of the input and the output. The temperature sensors measure a temperature of incoming and outgoing air. The heating element is regulated by the data obtained from the temperature sensors.

The heating device may comprise an ozone generator inside the housing. The ozone generator is effective for removing bad odours, or eliminating bacteria, viruses and mould. The ozone generator delivers ozone to the heated air stream. However, the ozone generator is optional and it has a separate start.

The heating device comprises a control unit inside the housing. The control unit may comprise a relay control, or a microcircuit control. The microcircuit control is more advanced compared to the relay control. The microcircuit control makes it possible to have a control that enables choosing different programs on a display, e.g. select temperature and time. The display may also show when the heating is running, when the targeted temperature has been reached, or when processing has ended.

The heating device comprises an overheating control that prevents the temperature to rise dangerously. Further, the heating device may comprise shields, such as grilles, on the mouths of the input and output in order to prevent touching hot surfaces.

The pouch is a flexible bag that comprises a closing member. The pouch may be flattened when it is not in use. The form of the flattened pouch may be any but e.g. a rectangular form is suitable for many kinds of items to be treated. The closing member may be a zipper. The closing member may be heat-resistant and airtight.

The pouch is made of a flexible or bendy material that is airtight. The pouch may be thermally insulated or non-insulated. The material may be formed of one layer only, or it may have different layers. The material may comprise an inner layer, a middle layer and an outer layer. The inner layer is the inside of the pouch and the outer layer is the outside of the pouch. The inner layer is heat-resistant and it may be made of silicone rubber or heat-resistant plastic, for instance. The above-mentioned materials may also be applied in solutions where there is only one layer.

The middle layer is optional and it is used when the pouch is thermally insulated. The middle layer may be made of cellular plastic, for example. The middle layer may be fireproof.

The outer layer is also optional. It may be made of some durable material, such as a fabric or reinforced plastic. The outer layer may be fireproof.

The pouch has an inlet and an outlet. The inlet of the pouch is connectable to the output of the heating device and the outlet of the pouch is connectable to the input of the heating device. The connections between the heating device and the pouch may be made by simple fittings, such as compression fittings, threaded connectors or clips that ensure an airtight connection between the heating device and the pouch.

When the apparatus is taken into use the inlet of the pouch is connected to the output of the heating device and the outlet of the pouch is connected to the input of the heating device. The item to be treated is put in the pouch and the closing member is closed. Air inside the apparatus is heated to a temperature that is from 55 to 65°C. The fan circulates heated air through the pouch and the heating device in such a manner that the pouch remains in a swollen state, i.e. there is overpressure inside the pouch. The overpressure, i.e. the positive pressure, helps warm air to penetrate into the items to be treated and prevents cold spots inside the pouch. The temperature is regulated by data obtained from the temperature sensors at the input and output. The temperature sensor at the input senses the temperature of the incoming air and sends data about it to the control unit. If there is a margin between the temperature of the incoming air and the temperature required in the pouch the heating element heats the air as much is required so that the temperature reaches a range from 55 to 65°C. The temperature of the outgoing air is measured at the output by the temperature sensor therein. The heating device is on through the whole treatment in order to make sure that there is always the prescribed temperature inside the bag and there is not cold spots where insect could survive. Further, air circulation by the fan decreases heating time of the pouch and thus improves energy efficiency of the apparatus. The treatment in the apparatus eliminates eggs, nymphs and adults.

It has been observed minimum temperatures and times to kill bedbugs. Adult bedbugs die at a temperature of 45°C in 90 minutes and at 48°C in 20 minutes. Eggs of bedbugs die at a temperature of 48°C in 90 minutes.

### BRIEF DESCRIPTION OF THE DRAWINGS

In the following the invention will be described in greater detail by means of preferred embodiments with reference to the attached drawings, in which
Figures 1 and 2 show perspective views of an apparatus;
Figure 3 shows a cross-directional view of a heating device;
Figure 4 shows a perspective view of a pouch;

### DETAILED DESCRIPTION OF THE INVENTION

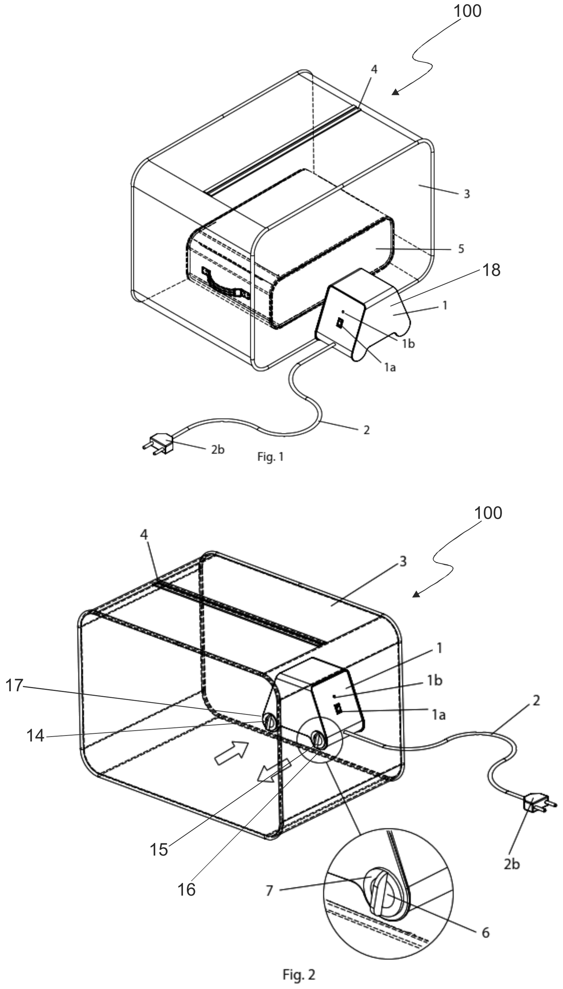

Figures 1 and 2 show perspective views of the apparatus 100 that is for eliminating bedbugs. The apparatus 100 comprises a heating device 1 and a pouch 3. The pouch 3 is shown as transparent in order to better understand the invention.

The heating device 1 comprises a housing 18 that is provided with a switch 1a. The housing 18 may be provided with an indicator light 1b showing that the heating device 1 is on. Instead of the indicator light 1b there may be a digital display. The heating device comprises a cable 2 and a plug 2b for connecting to mains electricity. The plug 2b is adapted to requirements in each country, e.g. for use in 220 - 240 V or 110 V.

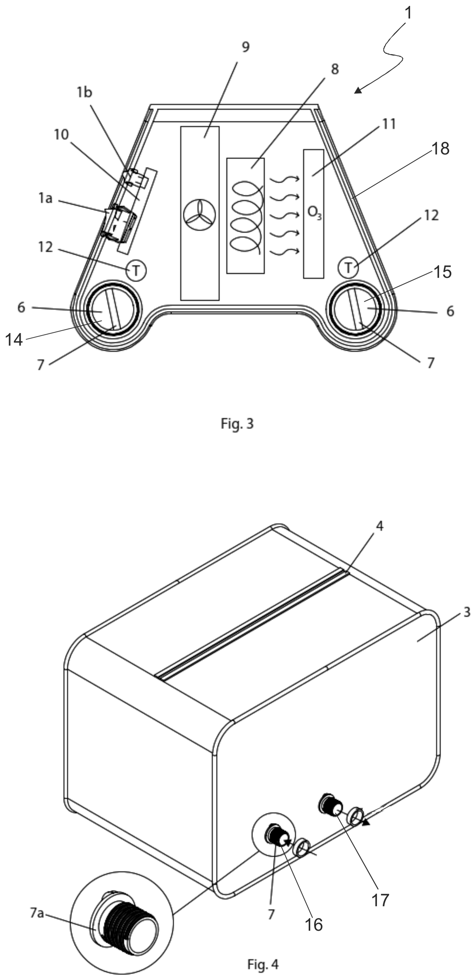

The heating device 1 comprises a heating element 8 and a fan 9 inside the housing as shown in Fig. 3. The heating device 1 has an input 14 and an output 15. The mouths 6 of the input 14 and the output 15 may comprise grilles for security reasons. The pouch 3 has an inlet 16 and an outlet 17. The inlet 16 of the pouch 3 is connectable to the output 15 of the heating device 1 and the outlet 17 of the pouch 3 is connectable to the input 14 of the heating device 1. In other words, during use of the apparatus 1 the inlet 16 of the pouch 3 is connected to the output 15 of the heating device 1 and the outlet 17 of the pouch 3 is connected to the input 14 of the heating device 1. After use the pouch 3 and the heating device 1 may be separated from one another.

The pouch 3 is an airtight flexible bag or sack that comprises a closing member 4, such as a zipper. The closing member 4 is airtight as well. The pouch 3 may have a layered structure, or it may have one layer only.

There is an item 5 to be treated inside the pouch 3. In this case the item 5 is a suitcase but the item 5 may be any other item that is suspected to be bedbug-infested. Heat penetrates to the item 5 within the heat treatment that may typically take from two to four hours.

Figure 3 shows a cross-directional view of the heating device 1. There are the heating element 8 and the fan 9 inside the housing 18. There may also be an ozone generator 11 but the ozone generator 11 is optional. If the ozone generator 11 is present safety precautions must be taken into consideration. The heating device 1 may have a separate switch to start ozonizing, or there may be a display wherefrom one must choose a program to be accomplished.

The order of the fan 9, the heating element 8 and the ozone generator 11 is preferably such that the fan 9 is the first in the flowing direction of the air, the heating element 8 is the second and the ozone generator 11 is the third if it is in the device 1.

The fan 9 circulates air that is heated by the heating element 8 through the pouch 3. The heated air enters to the pouch 3 through the inlet 16 and exits through the outlet 17. There may be a temperature sensor 12 in the vicinity of the input 14 and the output 15. The temperature sensors 12 measure a temperature of incoming and outgoing air. The heating element 8 is regulated by the data obtained from the temperature sensors 12.

A slight overpressure is created inside the pouch 3 in such a manner that the output 15 has greater cross-sectional area compared to the input 14. The slight overpressure keeps the flexible pouch 3 swollen so that the heated air reaches every inch of the item to be treated.

The heating device comprises a control unit 10 inside the housing 18. The control unit may comprise a relay control, or a microcircuit control. The control unit 10 is also an interface for the temperature sensors 12 and the switch 1a.

Figure 4 shows a perspective view of the pouch 3. The pouch 3 has an inlet 16 and an outlet 17. Connectors 7 are installed through the inside of the pouch 3 in such a manner that a connector 7 is screwed so that the material of the pouch 3 is between the flange 7a of the connector 7 and the housing 18 of the heating device 1. Thus, the input 14 and output 15 of the heating device 1 and the connectors 7 are provided with compatible threads. The connection between the heating device 1 and the pouch 3 may comprise gaskets.

It will be obvious to a person skilled in the art that, as the technology advances, the inventive concept can be implemented in various ways. The invention and its embodiments are not limited to the examples described above but may vary within the scope of the claims.

## Claims

1. An apparatus (100) for eliminating insects of genus Cimex, the apparatus (100) comprises a heating device (1) and a pouch (3) that are separate units, the heating device (1) comprising a heating element (8) and a fan (9) inside a housing (18), the heating device (1) has an input (14) and an output (15) and the pouch (3) has an inlet (16) and an outlet (17), the inlet (16) of the pouch (3) being connectable to the output (15) of the heating device (1) and the outlet (17) of the pouch (3) being connectable to the input (14) of the heating device (1).

2. The apparatus according to claim 1, **characterized in that** the output (15) of the heating device (1) has greater cross-sectional area than the input (14) of the heating device (1).

3. The apparatus according to claim 1 or 2, **characterized in that** the heating device comprises an ozone generator (11).

4. The apparatus according to any preceding claim, **characterized in that** the heating device (1) comprises one temperature sensor (12) at the input (14) and another temperature sensor (12) at the output (15).

5. The apparatus according to any preceding claim, **characterized in that** the heating device (1) comprises a power switch (1a) on the housing (18).

6. The apparatus according to any preceding claim, **characterized in that** the heating device (1) comprises an indicator light (1b) on the housing (18).

7. The apparatus according to any preceding claim 1 to 6, **characterized in that** the heating device (1) comprises a display on the housing (18).

8. The apparatus according to any preceding claim, **characterized in that** the heating device (1) comprises a control unit (10).

9. The apparatus according to claim 8, **characterized in that** the control unit (10) comprises relays.

10. The apparatus according to claim 8, **characterized in that** the control unit (10) comprises a microcircuit.

11. The apparatus according to any preceding claim, **characterized in that** the pouch (3) is collapsible.

12. The apparatus according to any preceding claim, **characterized in that** the pouch (3) is heat-insulated.

13. The apparatus according to any preceding claim, **characterized in that** the pouch (3) comprises a closing member.

14. The apparatus according to claim 13, **characterized in that** the closing member (3) is a zipper (4).

15. The apparatus according to any preceding claim, **characterized in that** each of the inlet (16) and outlet (17) are attached to the input (14) and output (15) of the heating device (1) by a threaded connector (7) comprising a flange (7a).
